# EUROPÄISCHE PATENTSCHRIFT

(11) **EP 2 614 045 B1**
(45) Veröffentlichungstag und Bekanntmachung des Hinweises auf die Patenterteilung: **18.11.2015**
(21) Anmeldenummer: 11769801.9
(22) Anmeldetag: 12.09.2011
(51) Int. Cl.: C07C 227/42, C07C 229/64, A61K 31/606, A61P 29/00

(54) **VERFAHREN ZUR HERSTELLUNG KRISTALLINER 5-AMINOSALICYLSÄURE**
METHOD FOR PRODUCING CRYSTALLINE 5-AMINOSALICYLIC ACID
PROCÉDÉ DE PRODUCTION D'ACIDE 5-AMINOSALICYLIQUE CRISTALLIN

(30) Priorität: 10.09.2010 EP 10176224
(43) Veröffentlichungstag der Anmeldung: 17.07.2013
(73) Patentinhaber: PharmaZell GmbH, 83064 Raubling (DE)
(72) Erfinder: GAAB, Stefan, 83022 Rosenheim (DE); HARTUNG, Alexander, 83512 Wasserburg (DE); AIGNER, Arno, 83104 Tuntenhausen (DE)
(74) Vertreter: Von Kreisler Selting Werner - Partnerschaft von Patentanwälten und Rechtsanwälten mbB
(86) Internationale Anmeldenummer: PCT/EP2011/065735
(87) Internationale Veröffentlichungsnummer: WO 2012/032185

(56) Entgegenhaltungen:
- EP-A1- 2 172 193
- WO-A1-01/96280
- WO-A1-2008/013416
- WO-A2-2004/093884
- US-A1- 2009 264 386
- HAGSTEN ET AL: "Identifying sources of batch to batch variation in processability", POWDER TECHNOLOGY, ELSEVIER SEQUOIA, LAUSANNE, CH, Bd. 183, Nr. 2, 18. März 2008 (2008-03-18) , Seiten 213-219, XP022541018, ISSN: 0032-5910, DOI: DOI:10.1016/J.POWTEC.2007.07.042
- SIROLA I ET AL: "Effect of crystal size and shape on bulk density of pharmaceutical powders", JOURNAL OF CRYSTAL GROWTH, ELSEVIER, AMSTERDAM, NL, vol. 181, no. 4, 1 November 1997 (1997-11-01), pages 403-409, XP004099528, ISSN: 0022-0248, DOI: 10.1016/S0022-0248(97)00299-6

## Beschreibung

### Gebiet der Erfindung

Die Erfindung bezieht sich auf ein Verfahren zur Herstellung von kristalliner 5-Aminosalicylsäure mit besonders hoher Schüttdichte.

### Hintergrund der Erfindung

Aminosalicylsäuren werden schon seit langem als Wirkstoffe in Medikamenten eingesetzt. So findet die 4-Aminosalicylsäure (*para*-Aminosalicylsäure, PAS; IUPAC: 5-Amino-2-hydroxybenzoesäure) seit den 1940er Jahren Anwendung als Antibiotikum in der Behandlung von Tuberkulose sowie als Medikament in der Behandlung entzündlicher Darmerkrankungen (inflammatory bowel diseases, IBD) wie Colitis Ulcerosa und Morbus Crohn. Es handelt sich bei der Colitis Ulcerosa um eine chronisch-rezidivierende Entzündung des Dickdarms mit unbekannter Ätiologie, die mit Hyperämie, Schwellung und Ulzerationen der Mukosa und Submukosa einhergeht. Es wird ein schubweiser oder kontinuierlich progredienter Verlauf beobachtet, der durch unvorhersehbare Verschlimmerungen und Remissionen charakterisiert ist. Der Morbus Crohn (Enteritis regionalis) ist ebenfalls eine chronische Entzündung unklarer Genese, welche aber den gesamten Darmbereich mit Hauptlokalisation im unteren Dünndarm und/oder Dickdarm betreffen kann. Typisch dabei ist ein segmentieller Befall, bei dem alle Wandschichten betroffen sind und es häufig zur Fistel- und Abszessbildung kommen kann.

Die 5-Aminosalicylsäure (5-ASA, Mesalazin oder auch Mesalamin genannt) wird zur Behandlung einer schwachen bis mittleren aktiven Colitis Ulcerosa eingesetzt.

Die pharmakologische Wirkung beruht dabei auf topischen Effekten mit der Darmmukosa.

Zur Erreichung der pharmakologischen Schutzwirkung wird die 5-ASA in Dosen von >3g/Tag verabreicht. Die 5-Aminosalicylsäure wird mit dem Ziel einer hohen Bioverfügbarkeit in verschiedenen Darreichungsformen als hochdosierte Formulierungen eingesetzt: dazu gehören Suppositorien, Enemas, Sachets mit Mikropellets und Tabletten. Besonders für die Herstellung hoch dosierter Formulierungen in Tablettenform eignet sich eine 5-Aminosalicylsäure mit hoher Schüttdichte.

In WO-A-01/96280 wird die Herstellung von 5-Aminosalicylsäure mittels eines elektrochemischen Verfahrens bei besonders niedrigen Temperaturen beschrieben, durch die Vorteile für die Herstellung erreicht werden konnten. Folgend auf einen elektrochemischen Schritt (electrochemical reduction) wird dabei ein Rohprodukt isoliert, das nach einem Reinigungsschritt zu einem kristallinen Reinprodukt, entsprechend den Vorgaben der Arzneibücher hinsichtlich der chemischen Qualität, umgewandelt wird. Entsprechend den Anforderungen an die jeweilige galenische Zubereitung erfolgt anschliessend die Umwandlung des Rohproduktes durch Kristallisation in das Reinprodukt unter verschiedenen Kristallisationsbedingungen.

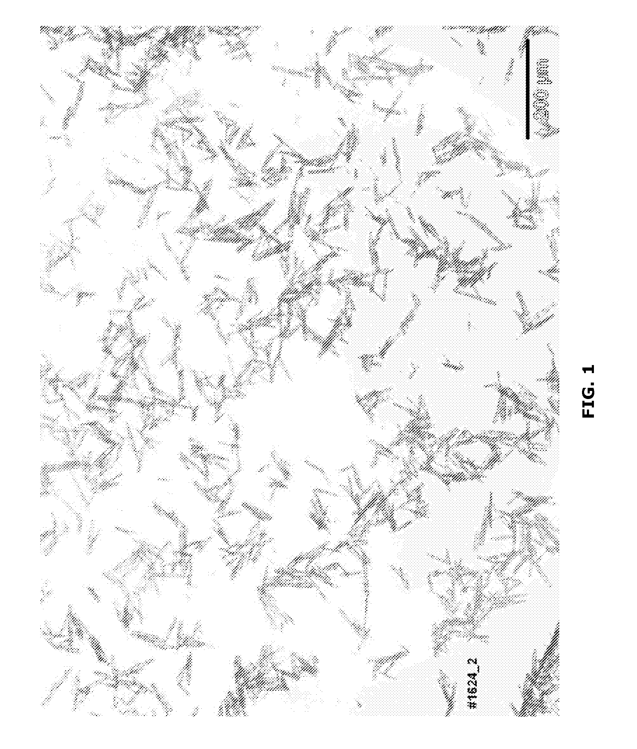

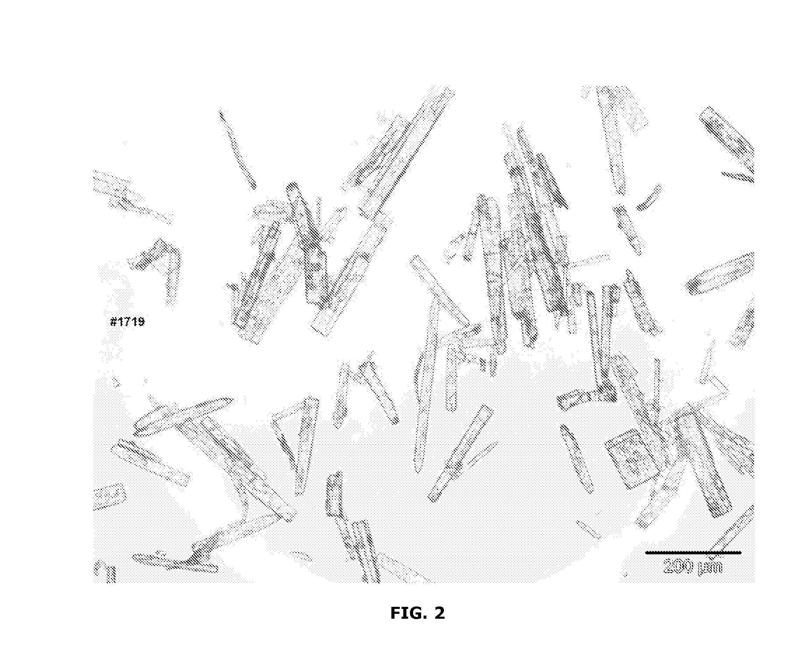

Nach dem Stand der Technik ist bekannt, dass neben der Fälldauer insbesondere die Temperatur und die Konzentration einen großen Einfluss auf die Bildung der Kristallgrößen haben. Die Anwendung unterschiedlicher Kristallisationsbedingungen führt folglich zu unterschiedlichen Kornverteilungen und unterschiedlich dichten Materialien. Beispielsweise werden bei niedriger Kristallisationstemperatur hauptsächlich feinere Kristalle, bei höherer Temperatur eher gröbere Kristalle erhalten. Dabei wachsen die großen Kristalle, während die kleinen Kristalle sich auflösen, wie es auch den Regeln für das Verhalten von Substanzen bei Kristallisationsvorgängen entspricht. Dieses ist gemäß dem Stand der Technik unter dem Lichtmikroskop nachweisbar (vgl. Fig. 1 und Fig. 2).

Bei der Kristallisation der 5-Aminosalicylsäure bilden sich unter verschiedenen Temperaturbedingungen grundsätzlich nadelförmige Kristalle in unterschiedlichen Kornverteilungen.

Beispielsweise werden bei niedrigen Temperaturen erwartungsgemäß feine Kristalle erhalten und bei hohen Temperaturen grobe Kristalle. Die Kristallisation kann aus einer wässrigen Lösung unter Zusatz in Höhe von 0 bis 100 Gew.% der wässrigen Lösung von ASA eines aprotischen oder protisch polaren, mit Wasser mischbaren Lösungsmittels erfolgen. Als Lösungsmittel können Aceton, Ethanol, Methanol oder iso-Propanol zum Einsatz kommen. Der anzuwendende Temperaturbereich liegt zwischen 25 °C und 150 °C.

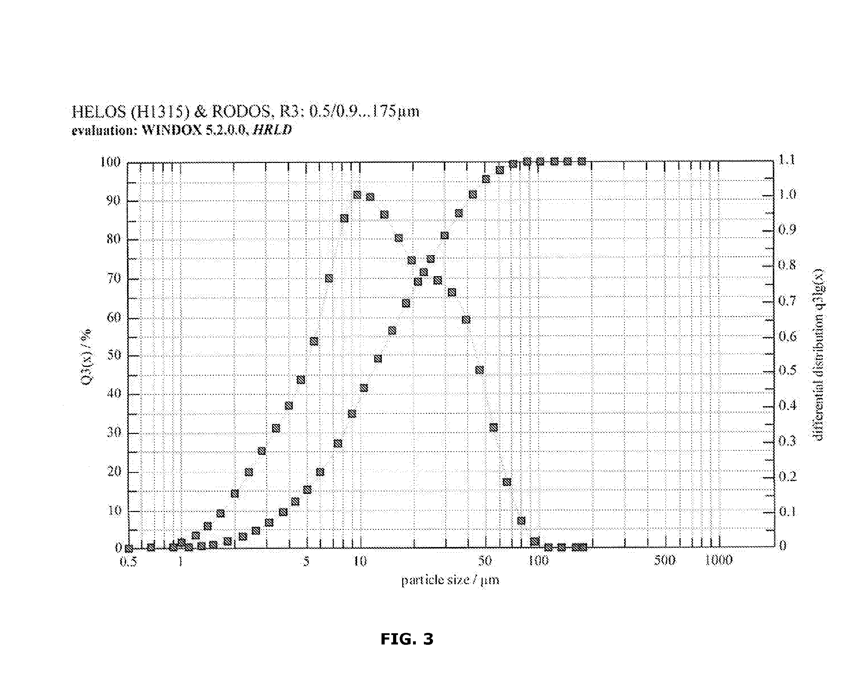

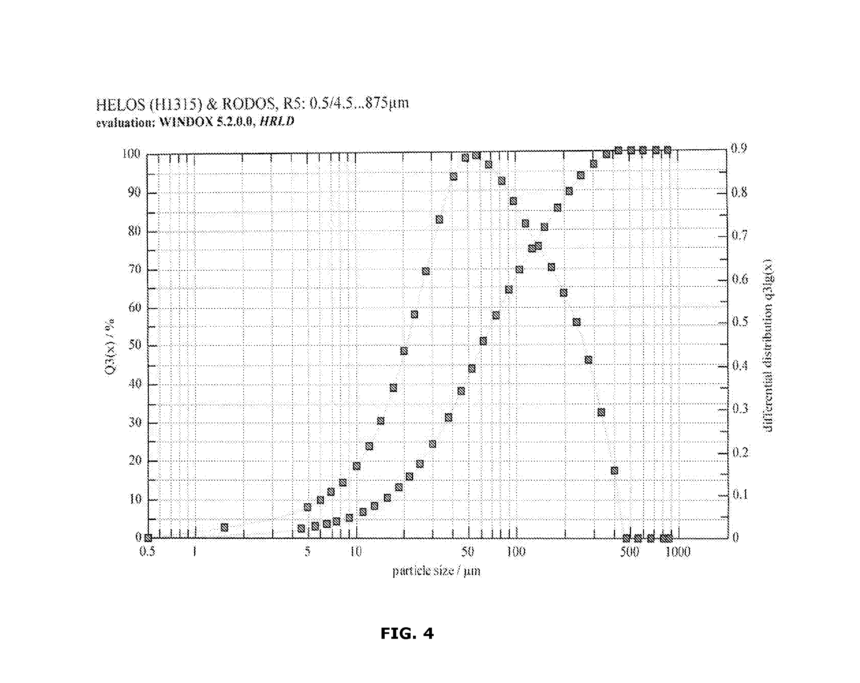

Die nadelförmigen Kristalle weisen im Allgemeinen eine geringe Schütt- und Stampfdichte auf, weil sich die Nadeln durch ihre Struktur bedingt verhaken und nicht ideal gepackt werden können, wie am Beispiel zweier nadelförmiger Kristallfraktionen mit unterschiedlicher Kornverteilung in den Fig. 3 und Fig. 4 gezeigt werden kann. Die Nadelstruktur verursacht auch ein schlechtes Fließverhalten, das für die galenische Verarbeitung Nachteile hat.

Hagsten et al.: "Identifying sources of batch to batch variation in processability", Powder Technology, Elsevier Sequoia, Lausanne, CH, Bd. 183, Nr. 2, 18. März 2008 (2008-03-18), Seiten 213-219, offenbart 5-ASA mit einer Pressdichte von bis zu 568 g/L (Tab. 1) hergestellt durch Extrusion. Pressdichte ist als Masse der Volumeneinheit eines Pulvers nach Anwendung eines bestimmten Pressdrucks definiert. Die verschiedenen hergestellten Chargen besitzen eine mittlere Korngröße von etwa 10 bis 14 µm. Außerdem ist die Verwendung als pharmazeutischer Wirkstoff offenbart.

WO 2004/093884 offenbart 5-ASA hergestellt durch Trockenvermahlung. Außerdem ist die Verwendung als pharmazeutischer Wirkstoff gegen Morbus Crohn und Colitis Ulcerosa offenbart.

EP 2172193 offenbart die Herstellung von 5-ASA (Mesalamine) durch die Herstellung einer Suspension und anschließender Nassvermahlung. Außerdem ist die Verwendung als pharmazeutischer Wirkstoff offenbart.

WO 2008/013416 offenbart die Herstellung von 5-ASA (Mesalazine) durch Dispergierung von 5-ASA in einem Lösungsmittel und anschließender Nassvermahlung. Außerdem ist die Verwendung als pharmazeutischer Wirkstoff offenbart.

Sirola I., et al: "Effect of crystal size and shape on bulk density of pharmaceutical powders", in Journal of Crystal Growth, Elsevier, Amsterdam, NL, Band 181, Nr. 4, 01. November 1997, Seiten 403-409 offenbart eine 5-ASA mit einer Kornverteilung (in µm, gemessen durch Lasterstrahlbeugung) von D₁₀ = 10,1, D₅₀ = 39,1 und D₉₀ = 86,0, sowie einer Schüttdichte von 0.530 g/mL.

### Kurzbeschreibung der Erfindung

Eine Aufgabe der vorliegenden Erfindung war es somit, ein Verfahren bereitzustellen, mit dem sich eine kristalline 5-ASA erhalten lässt, die hinsichtlich ihrer Schüttdichte den Anforderungen einer hohen Dosierbarkeit und Bioverfügbarkeit bei der Verwendung als Wirkstoff in entsprechenden Darreichungsformen genügt.

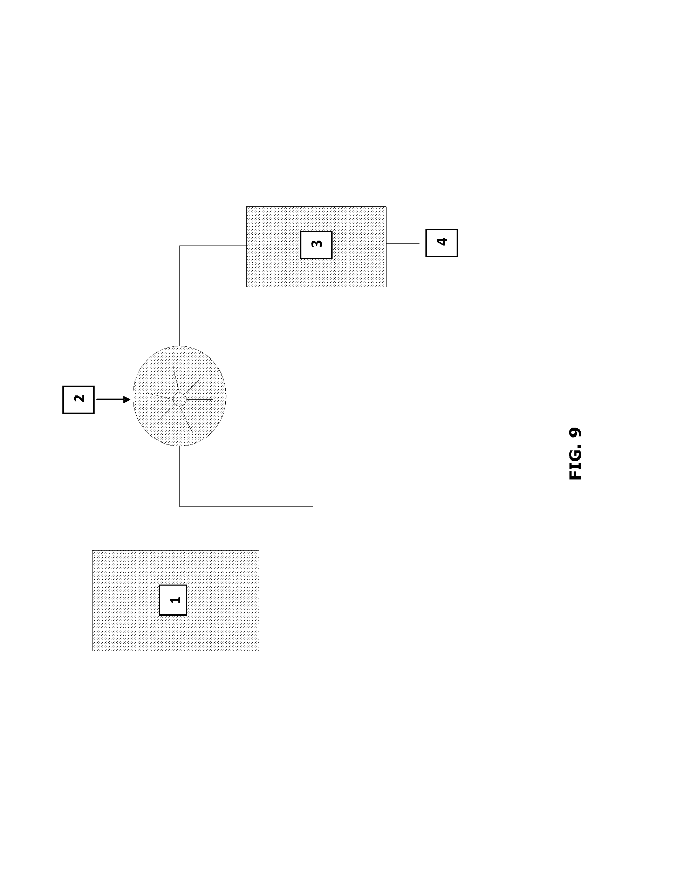

Es wurde nun bei der Kristallisation von 5-Aminosalicylsäure überraschend gefunden, dass ausgehend von einer 5-ASA mit grober Kristallstruktur durch einen Zerkleinerungsschritt, durch den das Breite- zu Länge-Verhältnis der Kristalle beeinflusst wird, Kristalle mit ungewöhnlich hohen Schüttdichten erhalten werden können. Dieser unvorhergesehene Effekt wird durch die Anpassung der Kristallisationstemperatur an die Konzentration des Substrates und die Fällzeiten zum Beispiel im Lösungsmittel Wasser in Kombination mit einem verfahrenstechnischen Zerkleinerungsschritt im Zustand der Suspension während oder nach beendeter Kristallisation einer geeigneten Ausgangsqualität erreicht (vgl. Fig. 9). Die Zerkleinerung kann wie in Fig. 9 gezeigt von einem Behälter in ein Vorlagegefäß erfolgen oder aber auch im Kreislauf durchgeführt werden, um den zusätzlichen Behälter einzusparen.

Die Einstellung der Kristallisationstemperatur begünstigt hauptsächlich das Längenwachstum der Kristalle. Erfindungsgemäß wird durch den Schritt der Nassvermahlung das Breite zu Länge-Verhältnis so eingestellt, dass ausgehend von einer besonders groben Partikelverteilung eine hohe Schüttdichte und/oder Stampfdichte erreicht werden kann. Dazu ist es erforderlich, ein grobes Ausgangsmaterial durch Einstellen der erforderlichen Kristallisationsparameter so zu erhalten, dass die Vermahlung den gewünschten Effekt zeigt.

Dementsprechend betrifft die vorliegende Erfindung das in den Patentansprüchen beschriebene Verfahren zur.Herstellung der 5-Aminosalicylsäure.

Die Erfindung umfasst somit die folgenden Punkte:
1. Verfahren zur Herstellung von kristalliner 5-Aminosalicylsäure (5-ASA) mit einer Schüttdichte von 300 g/L bis 700 g/L und einer Kornverteilung von X(10) = 1 µm- 30 µm, X(50) = 15 µm - 60 µm, X(90) = 35 µm - 220 µm umfassend die folgenden Schritte:
   (i) Kristallisieren von 5-ASA aus einer wässrigen Lösung mit oder ohne Zusatz eines aprotischen oder protisch polaren mit Wasser mischbaren Lösungsmittel im Konzentrationsbereich von 0 bis 100% w/w, bei einer Temperatur von 25 °C bis 150 °C und einem pH-Wert von 3,0 bis 5,0 unter Bildung einer Suspension von 5-ASA, und
   (ii) Nassvermahlen der Suspension in einem Homogenisator.
      1a. Verfahren nach Punkt 1, dadurch gekennzeichnet, dass die 5-ASA eine Stampfdichte von 510 g/L bis 900 g/L aufweist.
2. Verfahren nach Punkt 1, dadurch gekennzeichnet, dass das Kristallisieren bei einer Temperatur von 60 °C bis 120 °C erfolgt.
3. Verfahren nach Punkt 1, dadurch gekennzeichnet, dass das Kristallisieren bei einer Temperatur von 75 °C bis 115 °C erfolgt.
4. Verfahren nach Punkt 1, dadurch gekennzeichnet, dass das Kristallisieren bei einer Temperatur von 90 °C bis 110 °C erfolgt.
5. Verfahren nach irgendeinem der Punkte 1 bis 4, dadurch gekennzeichnet, dass der pH-Wert beim Kristallisieren 3,5 bis 4,5 beträgt.
6. Verfahren nach irgendeinem der Punkte 1 bis 5, dadurch gekennzeichnet, dass das protische Lösungsmittel Aceton, Methanol, Ethanol, iso-Propanol oder Gemische davon ist.
7. Verfahren nach irgendeinem der Punkte 1 bis 6, dadurch gekennzeichnet, dass bei der Nassvermahlung die Suspension eine Temperatur von <50 °C aufweist.
8. Verfahren nach irgendeinem der Punkte 1 bis 7, dadurch gekennzeichnet, dass die Nassvermahlung mittels einer Supraton- oder Ytron-Mühle bei Durchflussraten, die einem Gegendruck von 1 bis 10 bar entsprechen, durchgeführt wird.
9. Verfahren nach Punkt 8, dadurch gekennzeichnet, dass die Nassvermahlung bei Durchflussraten, die einem Gegendruck von 6 bis 9 bar entsprechen, durchgeführt wird.
10. Verfahren nach irgendeinem der Punkte 1 bis 9, dadurch gekennzeichnet, dass es einen weiteren Schritt (iii) des Abkühlens der Suspension umfasst.
11. Verfahren nach irgendeinem der Punkte 1 bis 10, dadurch gekennzeichnet, dass es einen weiteren Schritt (iv) des Trennens der 5-ASA Kristalle von der Mutterlauge umfasst.
12. Verfahren nach Punkt 11, dadurch gekennzeichnet, dass die Abtrennung mittels Zentrifugation erfolgt.
13. Verfahren nach irgendeinem der Punkte 1 bis 12, dadurch gekennzeichnet, dass es einen weiteren Schritt (v) des Trocknens der 5-ASA Kristalle umfasst.

Die durch das erfindungsgemäße Verfahren hergestellte 5-ASA ist gekennzeichnet durch die folgenden Punkte:
(i) durch eine Schüttdichte von 300 g/L bis 700 g/L, und eine Kornverteilung von X(10) = 1µm - 30 µm, X(50) = 15 µm - 60 µm, X(90) = 35 µm - 220 µm,
(ii) durch eine Stampfdichte von 510 g/L bis 900 g/L,
(iii) 5-Aminosalicylsäure (5-ASA) nach Punkt (i), gekennzeichnet durch eine Schüttdichte von 300 g/L bis 700 g/L, eine Stampfdichte von 510 g/L bis 900 g/L und eine Kornverteilung von X(10) = 1 µm - 30 µm, X(50) = 15 µm - 60 µm, X(90) = 35 µm - 220 µm.
(iv) 5-Aminosalicylsäure (5-ASA) nach Punkt (i), gekennzeichnet durch eine Schüttdichte von 300 g/L bis 400 g/L.
(v) 5-Aminosalicylsäure (5-ASA) nach Punkt (iii), gekennzeichnet durch eine Stampfdichte von 510 g/L bis 700 g/L.
(vi) 5-Aminosalicylsäure (5-ASA) nach Punkt (iii), gekennzeichnet durch eine Kornverteilung von X(10) = 3 µm - 20 µm, X(50) = 15 µm - 45 µm, X(90) = 50 µm - 100 µm, vorzugsweise durch eine Kornverteilung von X(10) = 3 µm - 5 µm, X(50) = 35 µm - 40 µm, X(90) = 90 µm - 100 µm.
(vii) 5-Aminosalicylsäure (5-ASA) nach Punkt (iii), gekennzeichnet durch eine Schüttdichte von 300 g/L bis 400 g/L, eine Stampfdichte von 510 g/L bis 700 g/L und eine Kornverteilung von X(10) = 3 µm - 20 µm, X(50) = 15 µm - 45 µm, X(90) = 50 µm - 100 µm, vorzugsweise durch eine Kornverteilung von X(10) = 3 µm - 5 µm, X(50) = 35 µm - 40 µm, X(90) = 90 µm - 100 µm.
(viii) 5-Aminosalicylsäure (5-ASA) nach Punkt (iii), gekennzeichnet durch eine Schüttdichte von 400 g/L bis 500 g/L.
(ix) 5-Aminosalicylsäure (5-ASA) nach Punkt (iii), gekennzeichnet durch eine Stampfdichte von 600 g/L bis 800 g/L.
(x) 5-Aminosalicylsäure (5-ASA) nach Punkt (iii), gekennzeichnet durch eine Schüttdichte von 400 g/L bis 500 g/L, vorzugsweise oberhalb 400 g/L bis 500 g/L, eine Stampfdichte von 600 g/L bis 800 g/L und eine Kornverteilung von X(10) = 5 µm - 25 µm, X(50) = 25 µm - 50 µm, X(90) = 50 µm - 200 µm, vorzugsweise durch eine Kornverteilung von X(10) = 7 µm - 10 µm, X(50) = 25 µm - 35 µm, X(90) = 80 µm - 90 µm. Offenbart sind ferner Verwendungen der durch das erfindungsgemäße Verfahren hergestellten 5-ASA: Suppositorien, Enemas, Sachets mit Mikropellets und Tabletten umfassend die 5-ASA gemäß irgendeinem der Punkte (i) bis (x), die Verwendung der 5-ASA gemäß irgendeinem der Punkte (i) bis (x) zur Herstellung von einer Darreichungsform ausgewählt aus der Gruppe bestehend aus Suppositorien, Enemas, Sachets mit Mikropellets und Tabletten, eine pharmazeutische Zusammensetzung umfassend die 5-ASA gemäß irgendeinem der Punkte (i) bis (x), sowie die 5-Aminosalicylsäure, wie in einem oder mehreren der Punkte (i) bis (x) definiert, zur Anwendung bei der Therapie und Prophylaxe einer Krankheit, die ausgewählt ist aus der Gruppe bestehend aus Morbus Crohn, Colitis Ulcerosa und Tuberkulose.

### Kurzbeschreibung der Figuren

Fig. 1 zeigt eine Lichtmikroskopieaufnahme von bei Temperaturen <40 °C erhaltener Kristalle von 5-Aminosalicylsäure
Fig. 2 zeigt eine Lichtmikroskopieaufnahme von bei Temperaturen >40 °C erhaltener Kristalle der gleichen Verbindung
Fig. 3 und Fig. 4 zeigen Beispiele von Teilchengrößenverteilungen zweier unterschiedlich großer nadelförmiger Kristallfraktionen, die nach dem Stand der Technik durch unterschiedliche Temperaturführung der Kristallisation mit unterschiedlicher Kornverteilung gemäß nachfolgender Tabelle 1 erhalten wurden:

**Tabelle 1: Beispiele von kristallinen 5-Aminosalicylsäuren mit unterschiedlichen physikalischen Charakteristika**

| Produkt | 5-ASA feine nadelförmige Kristalle | 5-ASA grobe nadelförmige Kristalle |
|---|---|---|
| Schüttdichte | 150 - 190 g/L | 250 - 270 g/L |
| Stampfdichte | 300 - 390 g/L | 450 - 480 g/L |
| Teilchengrößeverteilung | X (10) = 2 - 6 µm | X (10) = 10 - 15 µm |
| | X (50) = 8 - 20 µm | X (50) = 50 - 60 µm |
| | X (90) = 25 - 50 µm | X (90) = 200 - 220 µm |

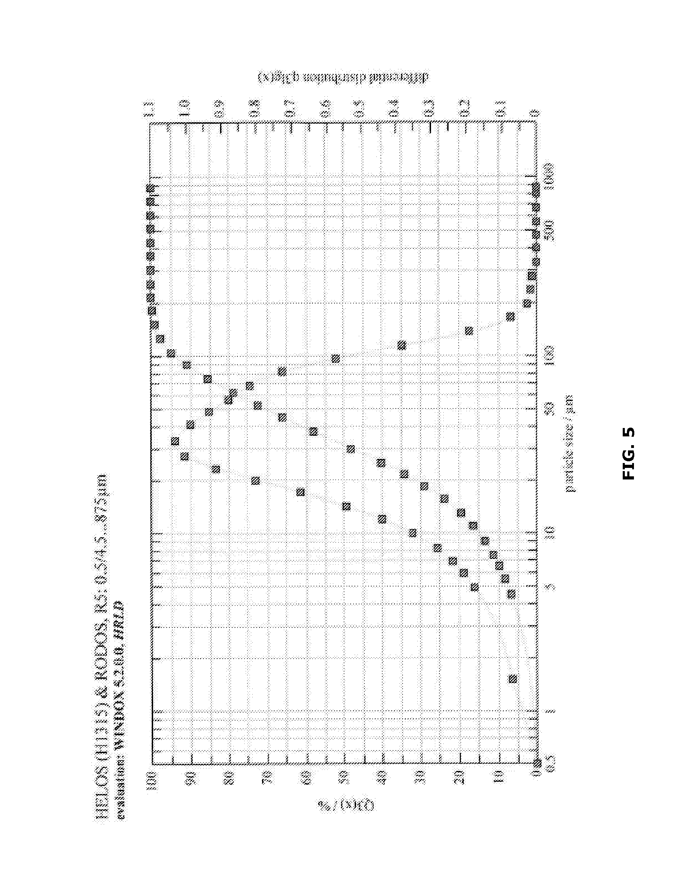

Fig. 5 zeigt die Teilchengrößenverteilung von 5-ASA mit hoher Schüttdichte

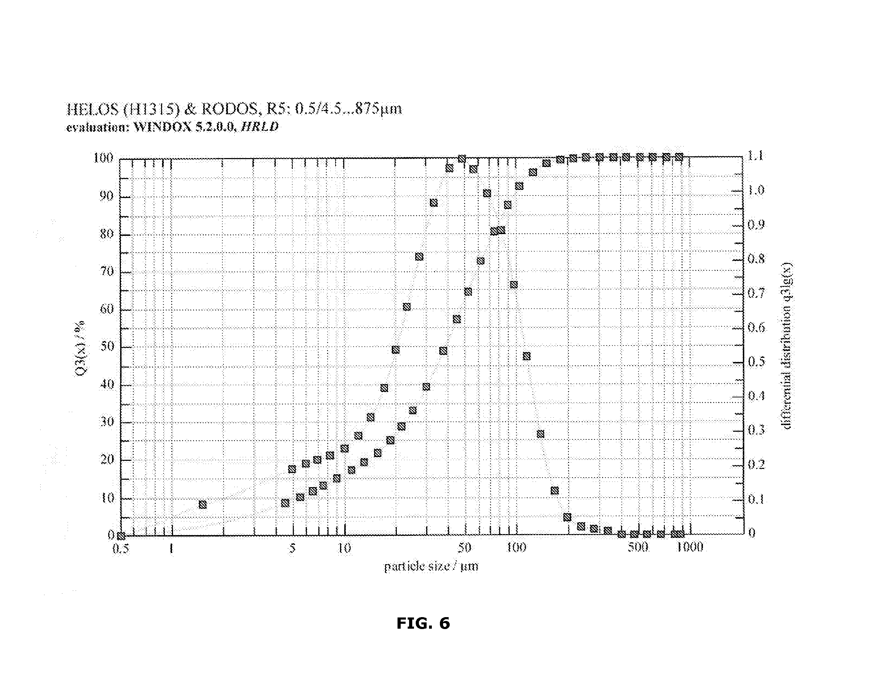

Fig. 6 zeigt die Teilchengrößenverteilung von 5-ASA mit extra hoher Schüttdichte

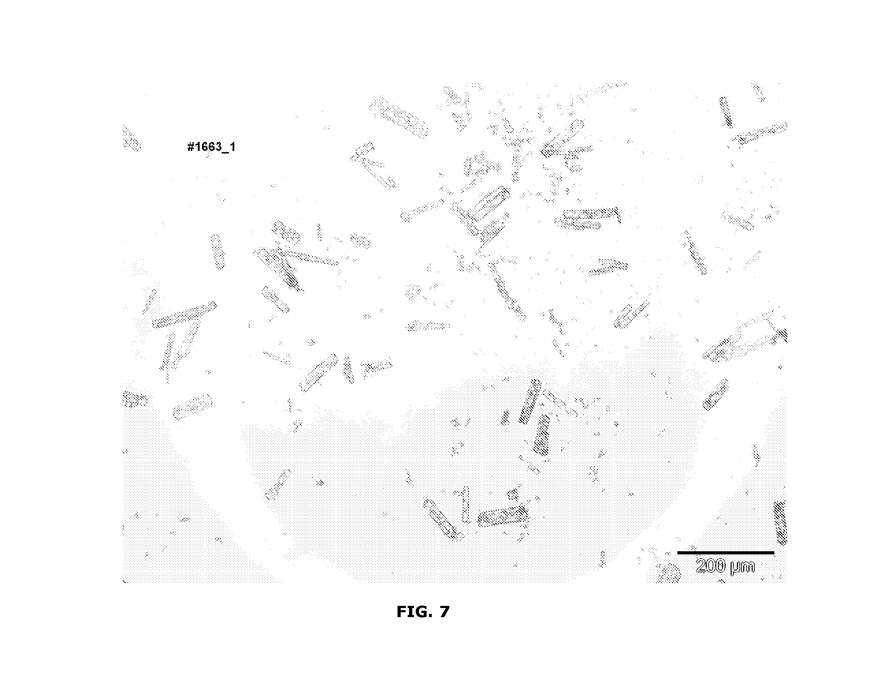

Fig. 7 zeigt eine Lichtmikroskopieaufnahme von 5-ASA mit hoher Schüttdichte

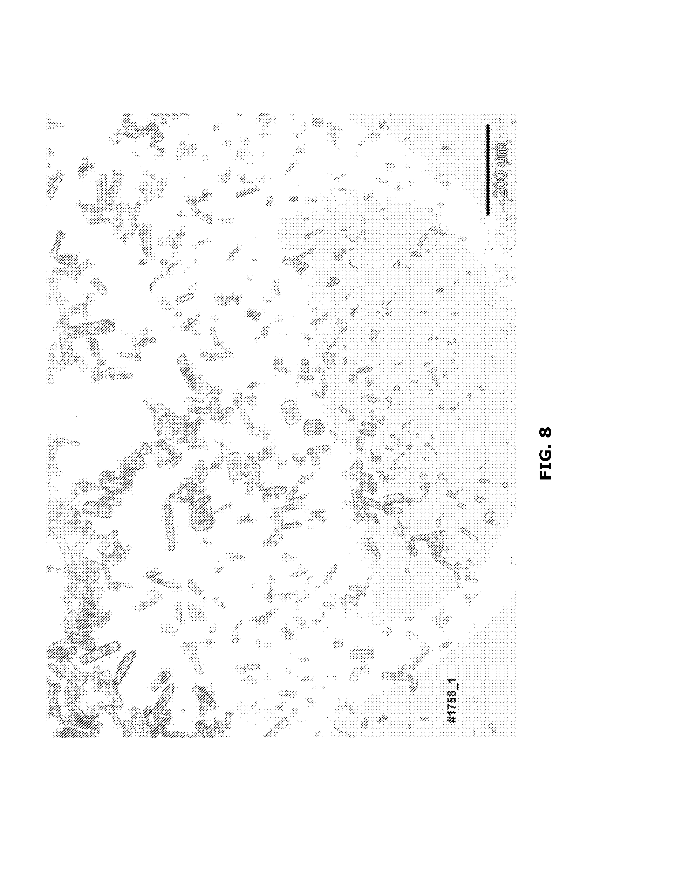

Fig. 8 zeigt eine Lichtmikroskopieaufnahme von 5-ASA mit extra hoher Schüttdichte
Fig. 9 zeigt eine schematische Darstellung der Nassvermahlung. Dabei bezeichnet 1: Kristallisationsbehälter, 2: Nassvermahlung, 3: Vorlagebehälter und 4: Zentrifuge/Trockner.

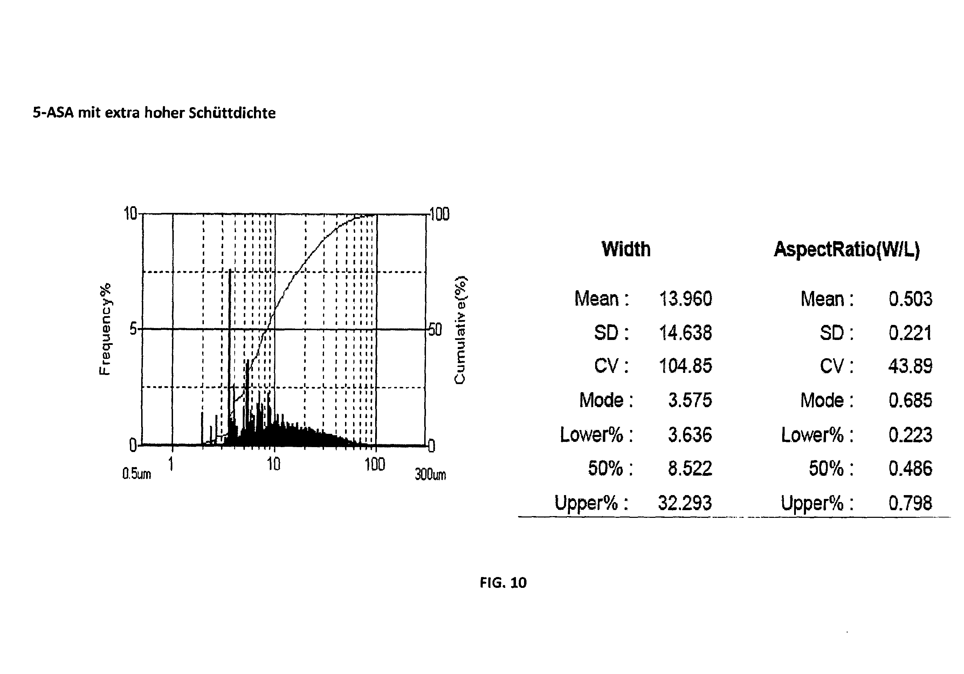

Fig. 10 zeigt das Laser-Diffraktogramm von 5-ASA mit extra hoher Schüttdichte.

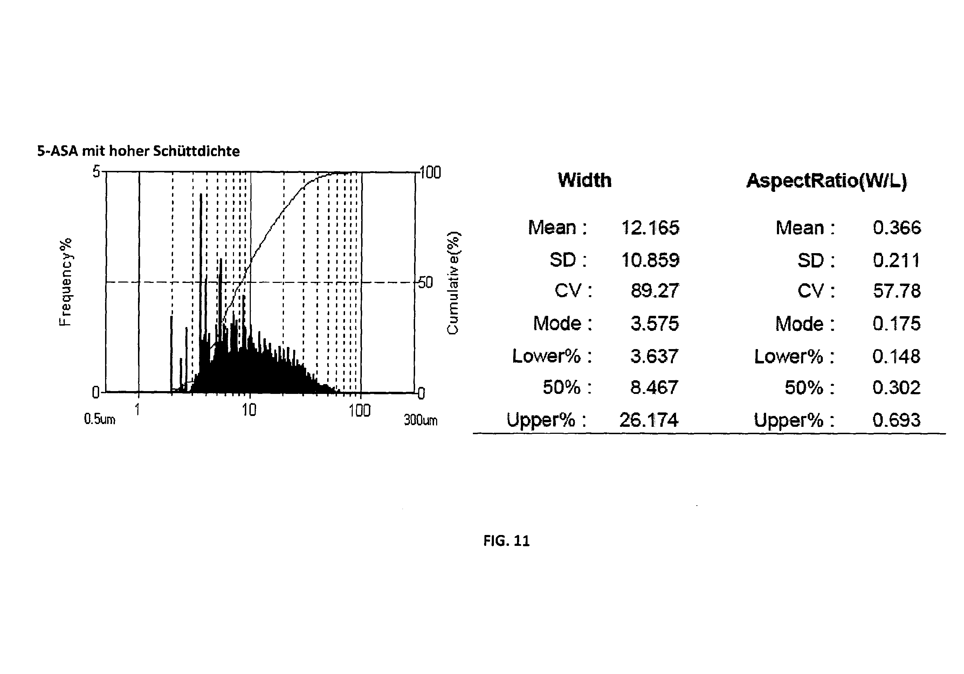

Fig. 11 zeigt das Laser-Diffraktogramm von 5-ASA mit hoher Schüttdichte.

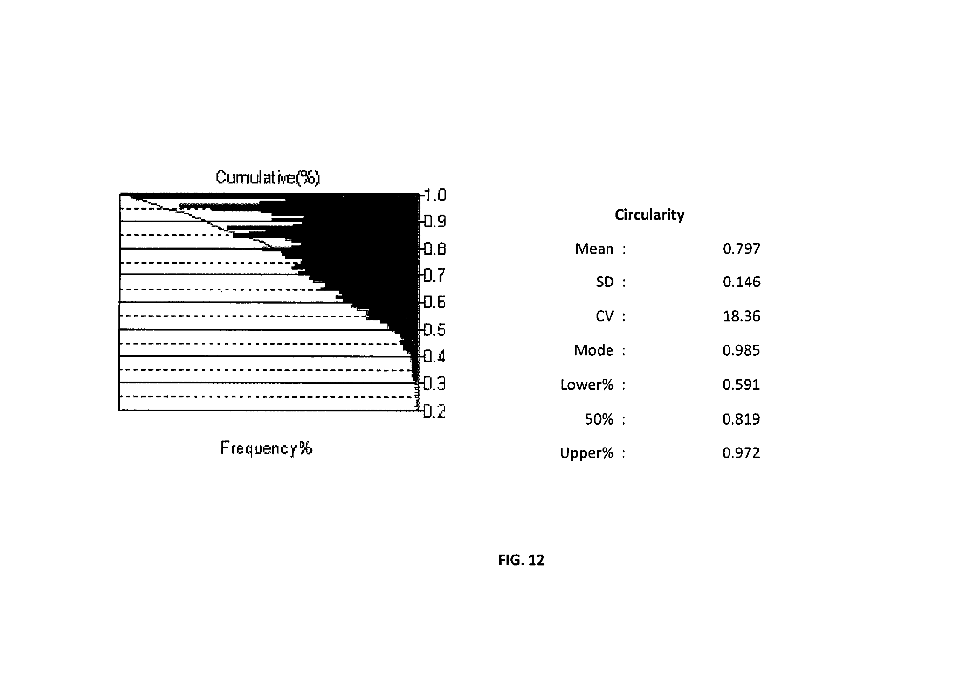

Fig. 12 zeigt die Zirkularität von 5-ASA mit extra hoher Dichte.

### Gegenstand der Erfindung

Ein Gegenstand der vorliegenden Erfindung ist daher ein Verfahren zur Herstellung von kristalliner 5-Aminosalicylsäure (5-ASA) mit einer Schüttdichte von 300 g/L bis 700 g/L und einer Kornverteilung von X(10) = 1 µm- 30 µm, X(50) = 15 µm - 60 µm, X(90) = 35 µm - 220 µm umfassend die folgenden Schritte:
(i) Kristallisieren von 5-ASA aus einer wässrigen Lösung von 5-ASA ohne oder unter Zusatz von protisch oder aprotisch polaren Lösungsmitteln im Konzentrationsbereich von 0 bis 100 % w/w bei einer Temperatur von 25 °C bis 150 °C und einem pH-Wert von 3,0 bis 5,0 unter Bildung einer Suspension von 5-ASA und (ii) Nassvermahlen der Suspension in einem Homogenisator.

In einer erfindungsgemäßen Ausführungsform wird der Schritt des Auskristallisierens der 5-ASA bei einer Temperatur von 60 °C bis 120 °C und in einer weiteren Ausführungsform bei einer Temperatur von 75 °C bis 115 °C sowie in einer noch weiteren Ausführungsform bei 90 bis 110 °C durchgeführt. In einer weiteren bevorzugten Ausführungsform beträgt der pH-Wert beim Kristallisieren 3 bis 4,5.

Das protisch polare Lösungsmittel ist ausgewählt aus der Gruppe bestehend aus verschiedenen mit Wasser mischbaren Alkoholen wie Methanol, Ethanol, Propanol, iso-Propanol, Butanol, iso-Butanol, sec-Butanol und tert-Butanol oder Gemischen davon. Bevorzugt ist/sind Methanol, Ethanol und iso-Propanol.

Das aprotisch polare Lösungsmittel ist ausgewählt aus der Gruppe bestehend aus mit Wasser mischbaren Ketonen wie Aceton, 2-Butanon, 2-Pentanon und 3-Pentanon oder Gemischen davon. Bevorzugt ist dabei Aceton.

Gemische von wenigstens einem protisch polaren und wenigstens einem aprotisch polarem Lösungsmittel können ebenfalls eingesetzt werden. Bevorzugt sind Gemische von Aceton, Methanol oder/und Ethanol.

Die Einstellung der Kristallisationstemperatur begünstigt hauptsächlich das Längenwachstum der Kristalle, wie oben gezeigt werden konnte. Durch die Nassvermahlung wird dann das Breite zu Länge-Verhältnis der Kristalle so eingestellt, dass ausgehend von einem groben Ausgangsmaterial, das noch nicht die gewünschten Eigenschaften zeigt, eine hohe Schüttdichte erreicht werden kann. Dazu ist es erforderlich, ein grobes Ausgangsmaterial durch Einstellen des erforderlichen Konzentrationsbereiches von 2 % bis 12 % w/w und eines Temperaturbereichs von 25 °C bis 150 °C zu erhalten, um währenddessen oder anschließend durch die Nassvermahlung den gewünschten Effekt zu erreichen. Bei steigenden Konzentrationen der zu kristallisierenden Lösung muss die Temperatur erhöht werden, um den erforderlichen Grobanteil zu erreichen.

Ein geeignetes grobes Ausgangsmaterial ist eine kristalline 5-ASA, wie sie unter den in den Beispielen 1 und 2 angegebenen Bedingungen der Zwischenschritte hergestellt wird und deren kristalline Eigenschaften ähnlich wie die der in Tabelle 1 aufgeführten grob kristallinen 5-ASA sind.

Die 5-ASA Kristalle enthaltende Suspension weist nach dem Schritt der Nassvermahlung in einer Ausgestaltung eine Temperatur von <150 °C auf, in einer weiteren Ausgestaltung von 25 bis 100 °C.

Die zur Durchführung des erfindungsgemäßen Verfahrens verwendeten Vorrichtungen zur Vermahlung können aus einem Rotor-Stator System aufgebaut sein, wobei der Rotor, der auch als das Werkzeug bezeichnet wird, verschiedene Formen und Ausführungen aufweisen kann.

Die Besonderheit des erfindungsgemäßen Nassvermahlungsschrittes besteht darin, dass dafür in diesem Prozess keine Mühlen im klassischen Sinne eingesetzt werden, sondern Apparate bzw. Vorrichtungen, die im Handel als Homogenisatoren erhältlich sind und durch die in überraschender Weise der erfindungsgemäße, gewünschte Effekt durch die Überlagerung verschiedener physikalischer Effekte erzielt werden kann, deren Einfluss in ihrer Gesamtheit nicht vorher berechnet oder anderweitig vorhergesagt werden kann.

Die Homogenisierungstechnik basiert auf der Anwendung der Hochdruckentspannung auf Flüssigkeiten, die die vordispergierten Partikel weiter zerkleinert. Das Ergebnis sind stabile Dispersionen für diverse Anwendungen. Das Produkt durchläuft die Hochdruckpumpe, wird verdichtet und anschließend im Homogenisierventil wieder entspannt. Der damit verbundene mechanische Energieeintrag bewirkt die gewünschten Produkteigenschaften.

Ohne an irgendeine Theorie gebunden sein zu wollen, kann vermutet werden, dass die physikalischen Effekte als Überlagerungen von mehrstufiger Scherungen in hydrodynamischen Scherfeldern, hochfrequenter oszillierender Kräfte, intensiver Durchmischung der flüssigen und festen Phase und einem Druckaufbau angesehen werden können.

Die Mahlparameter bei der Nassvermahlung können durch die Einstellung des Drucks zwischen 0 und 20 bar, beispielsweise von 4 bis 15 bar, so justiert werden, dass der gewünschte Effekt erzielt wird. Vorzugsweise können hierzu Supraton- oder Ytron-Homogenisatoren verwendet werden, aber auch andere im Handel erhältliche Homogenisatoren und inline Mühlen sind für die Zwecke der Erfindung geeignet.

Geeignete Homogenisatoren sind zum Beispiel ein Homogenisator der Ytron-Z-Baureihe, hergestellt von der Firma Ytron Process Technology GmbH & Co., KG (Bad Endorf, Deutschland, www.ytron.die) oder ein Homogenisator der Supraton-S-Baureihe, hergestellt von der BWS Technologie GmbH (Grevenbroich, Deutschland, www.supraton.com). Diese Homogenisatoren (Reaktoren) umfassen bis zu fünf, vorzugsweise bis zu drei Rotor/Stator-Sätze mit extrem geringem radialen Abstand. Eine bis mehrere flüssige Phasen sowie die darin suspendierten Stoffe werden in einer Zwangspassage durch das mehrreihige (5 oder 3 Reihen) Zahnkranz-Labyrinth geführt.

Die Nassvermahlung wird bevorzugt mittels eines Supraton- oder Ytron-Homogenisators bei Durchflussraten, die einem Gegendruck von 1 bis 20 bar, vorzugsweise von 4 bar bis 15 bar entsprechen, durchgeführt.

Bevorzugt als weitere, auf die Schritte (i) und (ii) folgende Schritte, sind (iii) das Kühlen der durch den Zerkleinerungsschritt erhaltenen Suspension auf Raumtemperatur (22 °C), ggf. (iv) das Trennen der Kristalle von der Mutterlauge, vorzugsweise durch Zentrifugation, sowie ggf. (v) das Trocknen des Produktes, beispielsweise in einem Schraubentrockner, vorzugsweise unter Vakuum bei < 1000 mbar und 80 °C. Durch das erfindungsgemäße Verfahren ist es möglich geworden, kristalline 5-ASA mit hohen Schütt- und/oder Stampfdichten zu erhalten.

Offenbart ist somit auch eine kristalline 5-Aminosalicylsäure, die eine Schüttdichte von 300 g/L - 700 g/L aufweist und die insbesondere nach dem hier beschriebenen erfindungsgemäßen Verfahren erhältlich ist.

Die erfindungsgemäß erhaltene 5-ASA weist eine Schüttdichte von 300 g/L - 700 g/L, in einer Ausgestaltung von 310 g/L - 600 g/L und in einer weiteren Ausgestaltung von 330 g/L - 500 g/L auf.

Die Stampfdichte der erfindungsgemäß erhaltenen 5-ASA kann im Bereich von 510 g/L-900 g/L, in einer Ausgestaltung im Bereich von 550 g/L - 800 g/L und in einer weiteren Ausgestaltung im Bereich von 600 g/L - 700 g/L liegen.

Die Korngrößenverteilung der erfindungsgemäß erhaltenen 5-ASA liegt in den Bereichen X(10) = 1 µm - 30 µm, X(50) = 15 µm - 60 µm, X(90) = 35 µm - 220 µm, in einer Ausgestaltung in den Bereichen X(10) = 3 µm - 20 µm, X(50) = 15 µm - 45 µm, X(90) = 50 µm - 100 µm, in einer weiteren Ausgestaltung X(10) = 5 µm - 25 µm, X(50) = 25 µm - 50 µm, X(90) = 50 µm - 200 µm, in einer weiteren Ausgestaltung in den Bereichen X(10) = 3 µm - 5 µm, X(50) = 35 µm - 40 µm, X(90) = 90 µm - 100 µm sowie in einer weiteren Ausgestaltung in den Bereichen X(10) = 7 µm - 10 µm, X(50) = 25 µm - 35 µm, X(90) = 80 µm - 90 µm.

Die Schüttdichte und die Stampfdichte werden dabei gemessen nach den dem Fachmann bekannten Methoden, wie sie in der USP ("United States Pharmacopeia") Monographie, z.B. USP 27, Vol. 1, S. 226 bis 227, May 1, 2009 - April 30, 2010, Schüttdichte 616, Methode 1, Stampfdichte 616, Methode 2, beschrieben sind. Die Messung der Kornverteilung erfolgt nach den Vorschriften der European Pharmacopeia, Supplement 6.6, Chapter 2.9.31 "Particle Size Analysis by Laser Light Diffraction" S. 5103 - 5107.

Offenbart ist somit eine kristalline 5-Aminosalicylsäure, gekennzeichnet durch eine Schüttdichte von 300 g/L bis 700 g/L, eine Stampfdichte von 510 g/L bis 900 g/L, und eine Kornverteilung X(10) = 1 µm - 30 µm, X(50) = 15 µm - 60 µm, X(90) = 35 µm - 220 µm.

Ferner offenbart ist eine kristalline 5-Aminosalicylsäure, gekennzeichnet durch eine Schüttdichte von 300 g/L bis 400 g/L, eine Stampfdichte von 510 g/L bis 700 g/L, und eine Kornverteilung X(10) = 3 µm - 5 µm, X(50) = 35 µm - 40 µm, X(90) = 90 µm - 100 µm, wie sie in Fig. 5 und Fig. 7 beispielhaft dargestellt ist.

Weiterhin offenbart ist eine kristalline 5-Aminosalicylsäure, gekennzeichnet durch eine Schüttdichte von 400 g/L bis 500 g/L, eine Stampfdichte von 600 g/L bis 800 g/L, und eine Kornverteilung X(10) = 7 µm - 10 µm, X(50) = 25 µm - 35 µm, X(90) = 80 µm - 90 µm, wie sie in Fig. 6 und Fig. 8 beispielhaft gezeigt ist.

Die Kristalle werden durch ihre Laserverteilung, das lichtmikroskopische Bild und das Breite zu Länge-Verhältnis beschrieben, die beispielsweise mittels der Bildanalyse mit Hilfe des Malvern Symex FPIA 3000 ermittelt werden können. Das Breite zu Länge-Verhältnis (B/L; Aspekt-Verhältnis) wird durch eine Verhältniszahl ausgedrückt. Im Falle der erfindungsgemäß erhaltenen 5-ASA mit extra hoher Schüttdichte liegt der Hauptanteil der Kristalle bei einem B/L-Verhältnis von 0,5. Daraus errechnet sich das Breite zu Länge-Verhältnis von B/L von 1:2. Bei der erfindungsgemäßen 5-ASA mit hoher Schüttdichte liegt das Breite zu Länge-Verhältnis bei 0,36 und entspricht damit einem Breite zu Länge-Verhältnis von 1:3. Die unterschiedlichen Breite zu Länge-Verhältnisse der erfindungsgemäß erhaltenen 5-ASA Kristallfraktionen mit hoher und extra hoher Schüttdichte bewirken die unterschiedlichen Schütt- und Stampfdichten sowie die unterschiedlich breiten Korngrößenverteilungen, die in den Laser-Diffraktogrammen gemäß Figur 10 und Figur 11 zu sehen sind.

Der Messwert der Zirkularität beschreibt die Abweichung der Kristallform von der idealen Kugelform. Der Wert von 1 entspricht einer idealen Kugel. Der Wert von fast 0,8 für die Zirkularität der erfindungsgemäß erhaltenen 5-ASA mit extra hoher Schüttdichte zeigt, dass die Form der Kristalle einer Kugelform wesentlich näher stehen als einer nadelförmigen Struktur. Dieses wiederum belegt die hohe Schütt- und Stampfdichte, die vergleichsweise enge Korngrößenverteilung und die daraus resultierende günstige galenische Verarbeitbarkeit, wie hier die Zirkularität der erfindungsgemäßen 5-ASA mit extrem hoher Dichte, gemessen mit Malvern Sysmex FPIA 3000 gemäß Figur 12.

Die 5-ASA Kristalle weisen in einer Ausführungsform ein Breite zu Länge-Verhältnis von 1:1,8 bis 1:2,2, in einer weiteren Ausführungsform von 1:2 auf.

Die 5-ASA Kristalle weisen in einer weiteren Ausführungsform eine Zirkularität von 0,7 bis 0,85 auf.

Einen weiteren Gegenstand der vorliegenden Offenbarung bilden pharmazeutische Darreichungsformen wie Suppositorien, Enemas, Sachets mit Mikropellets und Tabletten, umfassend die offenbarte kristalline 5-ASA.

Des Weiteren stellt die Verwendung der offenbarten kristallinen 5-ASA zur Herstellung von Suppositorien, Enemas, Sachets mit Mikropellets und Tabletten einen weiteren Gegenstand der vorliegenden Offenbarung dar.

Eine pharmazeutische Zusammensetzung, umfassend die offenbarte kristalline 5-ASA, bildet ebenfalls einen weiteren Gegenstand der vorliegenden Offenbarung.

Ein weiterer Gegenstand der vorliegenden Offenbarung ist kristalline 5-Aminosalicylsäure zur Anwendung in der Therapie und Prophylaxe einer Krankheit, die aus der Gruppe bestehend aus Morbus Crohn, Colitis Ulcerosa und Tuberkulose ausgewählt ist.

Die vorliegende Erfindung wird anhand der nachfolgenden Beispiele veranschaulicht, ohne jedoch darauf beschränkt zu sein.

### Beispiele

### Beispiel 1:

Etwa 600 kg Mesalazin (5-Aminosalicylsäure) roh werden in 2000 L Trinkwasser suspendiert und die Suspension bei etwa 30 °C mit Salzsäure auf pH <1 eingestellt. Dabei geht das Mesalazin als Hydrochlorid in Lösung. Die Lösung wird vorsorglich mit 17 kg Aktivkohle behandelt. Nach Abfiltrieren der Aktivkohle wird die klare Lösung bei 90 °C - 110 °C durch Zugabe von Lauge langsam auf einen pH-Wert von 3,5 bis 4 eingestellt. Dabei kristallisiert die 5-Aminosalicylsäure in groben Kristallen, die dann bei <50 °C entweder im Durchlauf oder im Kreislauf mittels eines geeigneten Homogenisators bei 10 bar im Zustand der Suspension vermahlen werden. Als Zerkleinerungswerkzeug dient ein 3-stufiger Supraton der Baureihe S 300.7.4 (Hersteller BWS Technologie, siehe oben), der ein dreiflügeliges Verteilerrad in der ersten Rotorstufe und eine Spaltbreite von 0,3 mm zwischen Rotoren und Statoren aufweist. Der Supraton wird mit einem Durchfluss von 13 bis 19 Tonnen/ Stunde und einer Leistungsaufnahme von 72 bis 85 Ah betrieben.

Charakteristika der Rotor- und Stator-Verhältnisse im Supraton:

| Rotor 1 / Stator 1 | Rotor 2 / Stator 2 | Rotor 3 / Stator 3 |
|---|---|---|
| dreiflügeliges Verteilerrad / Lochkreis 3mm | Schlitzbreite 7 mm / Lochkreis 2mm | Schlitzbreite 4 mm / Lochkreis 1mm |

Wahlweise kann ein Ytron-Homogenisator (Hersteller Ytron Process Technology, siehe oben) der Baureihe Z45.00 mit einem dreistufigen Rotor-Stator-Werkzeug mit einer Spaltbreite von 0,4 mm und Schlitzbreiten von Rotor- und Statorwerkzeugen von 1,5 mm mit dem gleichen Durchfluss wie der des oben angegebenen Supraton-Gerätes bei 45 Ah Leistungsaufnahme verwendet werden. Die Schlitzbreite des 3. Stators beträgt im verwendeten Ytron-Homogenisator 1 mm anstelle der 1,5 mm der Statoren 1 und 2.

Nach Vermahlung wird der pH-Wert der Suspension noch einmal überprüft und gegebenenfalls nachjustiert, die Suspension auf Raumtemperatur gekühlt und über eine Zentrifuge von der Mutterlauge getrennt und nachgewaschen. Das Produkt wird in einem Schraubentrockner im Vakuum [500 - 1000 mbar/ 40-80 °C] getrocknet. Die Ausbeute beträgt etwa 450 kg Reinprodukt.

### Beispiel 2:

Etwa 600 kg Mesalazin (5-Aminosalicylsäure) roh werden in 2000 L Trinkwasser suspendiert und die erhaltene Suspension wird bei etwa 30 °C mit Salzsäure auf pH <1 gestellt. Dabei geht das Mesalazin als Hydrochlorid in Lösung. Die Lösung wird vorsorglich mit 17 kg Aktivkohle behandelt. Nach Abfiltrieren der Aktivkohle wird die klare Lösung auf 70 °C - 110 °C, bevorzugt 80 °C bis 90 °C, geheizt und durch Zugabe von Lauge wird langsam der pH Wert von 3,5 bis 4 eingestellt. Dabei kristallisiert die 5-Aminosalicylsäure bei langsamer Rührerdrehzahl in etwas kleineren Kristallen als im Beispiel 1, die dann bei <50 °C entweder im Durchlauf oder im Kreislauf mittels eines wie oben beschriebenen Zerkleinerungswerkzeuges bei 10 bar in einer geeigneten Mühle im Zustand der Suspension vermahlen werden. Als Zerkleinerungswerkzeug werden die in Beispiel 1 beschriebenen Supraton- oder Ytron-Homogenisatoren verwendet.

Nach Vermahlung wird der pH-Wert noch einmal überprüft und gegebenenfalls nachjustiert, die Suspension auf Raumtemperatur gekühlt und über eine Zentrifuge von der Mutterlauge getrennt und nachgewaschen. Das Produkt wird in einem Schraubentrockner im Vakuum [500 - 1000 mbar/ 40-80 °C] getrocknet. Die Ausbeute beträgt etwa 450 kg Reinprodukt.

Die Eigenschaften der in den Beispielen 1 und 2 erhaltenen kristallinen 5-Aminosalicylsäuren werden in Tabelle 2 denjenigen von entsprechenden handelsüblichen Produkten (feinkristalline 5-ASA und grobkristalline 5-ASA) gegenübergestellt.

**Tabelle 2: Gegenüberstellung verschiedener kristalliner 5-Aminosalicylsäuren**

| Produkt | 5-ASA feine Kristalle^{*)} | 5-ASA grobe Kristalle^{*)} | Beispiel 1 | Beispiel 2 |
|---|---|---|---|---|
| Schüttdichte [g/L] | 150 - 190 | 250 - 270 | 400 - 500 | 300 - 400 |
| Stampfdichte [g/L] | 300 - 390 | 450 - 480 | 600 - 800 | 510 - 700 |
| Korngrößenverteilung [µm] | X(10) = 2-6 | X(10) = 10 -15 | X(10) = 7 - 10 | X(10)=3 - 5 |
| | X(50) = 8-20 | X(50) = 50 - 60 | X(50) = 25 - 35 | X(50)= 15 - 45 |
| | X(90) = 25-50 | X(90) = 200 - 220 | X(90) = 80 - 90 | X(90)= 90-100 |

| | | | | |
|---|---|---|---|---|
| ^{*)}.5-ASA feinkristallin und 5-ASA grobkristallin sind Vergleichsprodukte gemäß dem Stand der Technik | | | | |

Wie aus Tabelle 2 ersichtlich, weisen die nach dem erfindungsgemäßen Verfahren hergestellten erfindungsgemäßen kristallinen 5-Aminosalicylsäuren höhere Stampf- und Schüttdichten auf, als die bisher zur Verfügung stehenden kristallinen 5-Aminosalicylsäuren 5-ASA feinkristallin und 5-ASA grobkristallin gemäß dem Stand der Technik. Die erfindungsgemäßen kristallinen 5-Aminosalicylsäuren sind somit ausgezeichnet zur Herstellung von Medikamenten, insbesondere in Tablettenform, mit hoher Wirkstoffkonzentration geeignet.

### Gewerbliche Anwendbarkeit

Die Erfindung ermöglicht die Herstellung von 5-Acetylsalicylsäure mit besonders hoher Schüttdichte, was hohe Dosierungen und Bioverfügbarkeiten als Wirkstoff in entsprechenden Darreichungsformen, insbesondere in Form von Tabletten, ermöglicht.

## Patentansprüche

1. Verfahren zur Herstellung einer kristallinen 5-Aminosalicylsäure (5-ASA), die eine Schüttdichte von 300 g/L bis 700 g/L und eine Kornverteilung von X(10) = 1 µm - 30 µm, X(50) = 15 µm - 60 µm, X(90) = 35 µm - 220 µm aufweist, umfassend die folgenden Schritte:
(i) Kristallisieren von 5-ASA aus einer wässrigen Lösung von 5-ASA ohne oder unter Zusatz von protisch oder aprotisch polaren Lösungsmitteln im Konzentrationsbereich von 0 bis 100 % bei einer Temperatur von 25 °C bis 150 °C und einem pH-Wert von 3,0 bis 5,0 unter Bildung einer Suspension von 5-ASA, und
(ii) Nassvermahlen der Suspension in einem Homogenisator.

2. Verfahren nach Anspruch 1, **dadurch gekennzeichnet, dass** die 5-ASA eine Stampfdichte von 510 g/L bis 900 g/L aufweist.

3. Verfahren nach Anspruch 1 oder2, **dadurch gekennzeichnet, dass** das Kristallisieren bei einer Temperatur von 60 °C bis 120 °C erfolgt.

4. Verfahren nach irgendeinem der Ansprüche 1 bis 3, **dadurch gekennzeichnet, dass** es einen weiteren Schritt (iii) des Abkühlens der Suspension umfasst.

5. Verfahren nach irgendeinem der Ansprüche 1 bis 4, **dadurch gekennzeichnet, dass** es einen weiteren Schritt (iv) des Trennens der 5-ASA Kristalle von der Mutterlauge umfasst.

6. Verfahren nach irgendeinem der Ansprüche 1 bis 5, **dadurch gekennzeichnet, dass** es einen weiteren Schritt (v) des Trocknens der 5-ASA Kristalle umfasst.

## Claims

1. A process for producing a crystalline 5-aminosalicylic acid (5-ASA) having a bulk density of from 300 g/l to 700 g/l and grain size distribution of X(10) = 1 µm - 30 µm, X(50) = 15 µm - 60 µm, X(90) = 35 µm - 220 µm, comprising the following steps:
(i) crystallizing 5-ASA from an aqueous solution of 5-ASA with or without the addition of protic or aprotic polar solvents in a concentration range of from 0 to 100% at a temperature of from 25 °C to 150 °C and at a pH-value of from 3.0 to 5.0 to form a suspension of 5-ASA; and
(ii) wet grinding the suspension in a homogenizer.

2. The process according to claim 1, **characterized in that** said 5-ASA has a tapped density of from 510 g/l to 900 g/l.

3. The process according to claim 1 or 2, **characterized in that** said crystallizing is effected at a temperature of from 60 °C to 120 °C.

4. The process according to any of claims 1 to 3, **characterized by** comprising a further step (iii) of cooling the suspension.

5. The process according to any of claims 1 to 4, **characterized by** comprising a further step (iv) of separating the 5-ASA crystals from the mother liquor.

6. The process according to any of claims 1 to 5, **characterized by** comprising a further step (v) of drying the 5-ASA crystals.

## Revendications

1. Procédé de fabrication d'un acide 5-aminosalicylique (5-ASA) cristallin, qui présente une densité apparente en vrac comprise entre 300 g/l et 700 g/l et une granulométrie de X(10) = 1 µm à 30 µm, X(50) = 15 µm à 60 µm, X(90) = 35 µm à 220 µm, comprenant les étapes suivantes :
(i) cristallisation du 5-ASA à partir d'une solution aqueuse de 5-ASA avec ou sans ajout de solvants polaires protiques ou aprotiques dans une plage de concentration de 0 à 100 %, à une température comprise entre 25 °C et 150 °C et à une valeur de pH comprise entre 3,0 et 5,0, en formant une suspension de 5-ASA, et
(ii) broyage par voie humide de la suspension dans un homogénéisateur.

2. Procédé selon la revendication 1, **caractérisé en ce que** le 5-ASA présente une densité après compactage comprise entre 510 g/l et 900 g/l.

3. Procédé selon la revendication 1 ou 2, **caractérisé en ce que** la cristallisation se fait à une température comprise entre 60 °C et 120 °C.

4. Procédé selon l'une quelconque des revendications 1 à 3, **caractérisé en ce qu'**il comprend une étape supplémentaire (iii) de refroidissement de la suspension.

5. Procédé selon l'une quelconque des revendications 1 à 4, **caractérisé en ce qu'**il comprend une étape supplémentaire (iv) de séparation des cristaux de 5-ASA de la liqueur mère.

6. Procédé selon l'une quelconque des revendications 1 à 5, **caractérisé en ce qu'**il comprend une autre étape (v) de séchage des cristaux de 5-ASA.
